# EUROPEAN PATENT SPECIFICATION

(11) **EP 2 943 167 B1**
(45) Date of publication and mention of the grant of the patent: **03.04.2019**
(21) Application number: 13811360.0
(22) Date of filing: 18.12.2013
(51) Int. Cl.: A61F 5/02

(54) **BACK SUPPORT**
RÜCKENSTÜTZE
SUPPORT DORSAL

(30) Priority: 09.01.2013 DK 201370008
(43) Date of publication of application: 18.11.2015
(73) Proprietor: V V/Anja C Seiler Hansen, 5500 Middelfart (DK)
(72) Inventor: HANSEN, Anja Camilla Seiler, 5500 Middelfart (DK)
(74) Representative: Patentgruppen A/S
(86) International application number: PCT/DK2013/050440
(87) International publication number: WO 2014/108132

(56) References cited:
- US-A- 1 250 407
- US-A- 5 334 134
- US-A- 6 066 108
- US-A1- 2010 217 167

## Description

### Field of the invention

The present invention relates to the field of back supports.

### Background of the invention

There may be various reasons for a back support may be relevant. For instance it may be in relation to merely adjusting posture or for comfort reasons. In other instances it may be relevant in relation to back damages and back problems in general. In a situation where a person severely injures his back, e.g. a broken back, in order to recover it is necessary to provide a satisfying support for the back for at least a number of months to let the body's own restoring process occur unhindered.

An offer from the health care system in Denmark in this regard is a rigid back support mounted around the torso of the patient, on the back with one plate stretching from the pelvis to approximately halfway up the back, and on the front with three plates rigidly connected; a first stretching out over the thorax, a second stretching out over the abdomen and a third stretching out over the upper part of the pelvis.

As this back support is rigid and stretches out over the main part of the front and back of the torso, it is really uncomfortable to wear. For instance, it impedes breathing and eating due to the constant pressure exerted on the abdomen and thorax. Furthermore, it is difficult to handle and very difficult to clean. From the patent application US 2010/0217167 A1 it is known to form a back support with a belt to be tied around the user. However, this device exhibits much of the same disadvantages as described above. And from US 1,250,407 it is known to provide a device for providing abdominal support with leg straps. However this device does not provide substantial back support.

It is an object of the invention to provide an improved back support.

### Summary of the invention

The invention relates to a back support comprising a waist band with at least one front-facing fixing region and at least one back-facing supporting plate attached to said waist band; said at least one supporting plate being adapted to support the lower back region of the user. The back support is characterized in that it further comprises at least two first leg straps fixed in both ends unto said waist band in said fixing region; said first leg straps each forming a loop extending in use from the fixing region downwardly and behind a respective leg of the user and again upwardly to the fixing region;

The back support further comprises at least two second leg straps fixed in both ends unto said waist band in said fixing region; said second leg straps each forming a loop extending in use from the fixing region downwardly and behind a respective leg of the user and again upwardly to the fixing region; wherein said at least two first leg straps are adapted for, when in use, tightening behind the upper region of the thigh of the user, and said at least two second leg straps are adapted for, when in use, tightening behind the lower region of the thigh of the user.

With the present invention the before-mentioned disadvantages with the prior art has been overcome. Like in the prior art, the back support according to embodiments of the present invention provides a pressure exerted against at least the lower part of the user's spine, thereby providing a desired support. However, contrary to the prior art, the back support according to embodiments of the present invention is very comfortable to wear i.e. in that pressure exerted on the thorax may be avoided and pressure on the abdomen may be substantially lessened as compared to the prior art back support. These advantages may be observed for both sitting and standing positions of the user.

With two leg straps on each thigh of the user, a very satisfying embodiment is obtained. With an ideal adjustment of the length of each strap, a satisfying pressure to the back is believed to be possible.

In an embodiment of the invention, said supporting plate is substantially shaped as the shape of a back of a user to increase the comfortability for the user.

According to some embodiments, the supporting plate is shaped to touch the back over a wide area, such that a large pressure can be applied with only very little annoyance to the user.

In an embodiment of the invention, said supporting plate comprises a pad to increase the comfortability for the user.

According to some embodiments, the supporting plate may include or be formed of a relative soft material for comfort reasons. In some embodiments the supporting plate may comprise a pad formed of one or more suitable materials, e.g. a textile webbing sleeve having a filling of a suitable resilient rubber material, a gel or a silicone rubber. According to various embodiments, the supporting plate and/or an optional pad may at least comprise a material such as leather, plastic, fabric, elastic material, etc. A skilled person will be able to vary within common suitable supporting materials.

In an embodiment of the invention, said supporting plate comprises means for actively providing heating, cooling, and/or electrical impulses.

For some back patients, cooling or heating may be very pleasant and helpful. Furthermore, electrodes may provide electrical impulses, also helpful to some users.

In an embodiment of the invention, said waist band comprises a buckle for adjusting the circumference of the waist band.

The waist band should be adjustable to fit the user and furthermore to enable tightening to a desirable degree.

In an embodiment of the invention, said straps comprises buckles for adjusting the length of said straps.

The straps should be adjustable to enable tightening to a desirable degree to secure a sufficient pulling force to the back support.

In an embodiment of the invention, said back support further comprises a connecting means for connecting said waist band to a fixed structure in front of the user, preferably said connecting means comprises a hook, a snap hook and/or a strap comprising a loop.

The present invention may be ideally suitable for travelling on e.g. busses, trains, airplanes etc. Normally such travelling may be problematic and uncomfortable for people with a bad back; however, with the present invention it may be even highly comfortable to be on public transportation. When seated, the full pressure exerted on the back may then be provided by the seat in front of the user. Preferably the connecting means should comprise a length adjusting mechanism in order to fit any distance to a front seat.

In an embodiment of the invention, said back support further comprises a lower strap system for, when in use, assisting said second leg straps in maintaining their position on the lower region of the thigh of the user; said lower strap system comprising at least one strap surrounding the leg of said user.

In an embodiment of the invention, said lower strap system, when in use, comprises two straps on each leg of said user, one above the knee and one below the knee, both surrounding the legs, and at least one connecting strap for connecting said straps on each leg.

In an embodiment of the invention, said back support further comprises an upper strap system for, when in use, assisting waist band in maintaining its position around the waist; said upper strap system comprising at least one strap extending around the shoulder of the user.

Further to the already described straps, in some embodiments it may be advantageous to include straps supported over the shoulders of the user.

In an embodiment of the invention, said back support further comprises an upper strap system for, when in use, assisting waist band in maintaining its position around the waist;

In an embodiment of the invention, the straps are formed of an elastic material.

The straps may be provided in elastic materials, varying in width, elasticity, strength, flexibility and carrying capacity. Typical widths may be between 1 and 10 cm, such as between 3 and 7 cm. The elastic material may be any within the art commonly used stretchable fabric or elastomer.

In an embodiment of the invention, said at least one back-facing supporting plate has an area of more than 100cm², preferably more than 200cm².

The area of the supporting plate may vary in various embodiments, according to the need or desire of the user. In some embodiments the area of the supporting plate is adjustable.

In an embodiment of the invention, said at least one back-facing supporting plate is fixed on said waist band or said waist band is connected to said supporting plate in each side of said supporting plate.

### The figures

The invention will now be described with reference to the drawings of which
fig. 1 illustrates a back support according to an embodiment of the invention seen from the back,
fig. 2 illustrates the same embodiment as in fig. 1 seen from the front,
fig. 3 illustrates a prior art back support seen from the back, and
fig. 4 illustrates the same prior art back support seen from the front.

### Detailed description

The term "buckle" is intended to broadly cover standard tightening mechanisms such as Velcro and mechanisms commonly known from e.g. sports bags and seat belts.

The term "front-facing fixing region" is intended to cover the part of the waist band in the front used for fixing. This fixing can occur in a number of different suitable well-known ways, typically by some kind of buckles, and thus the size of the fixing region on the waist band may vary as well. However, in preferred embodiments the fixing region has a horizontal extension of less than 30 cm, such as less than 20 cm, centered on the navel of the user.

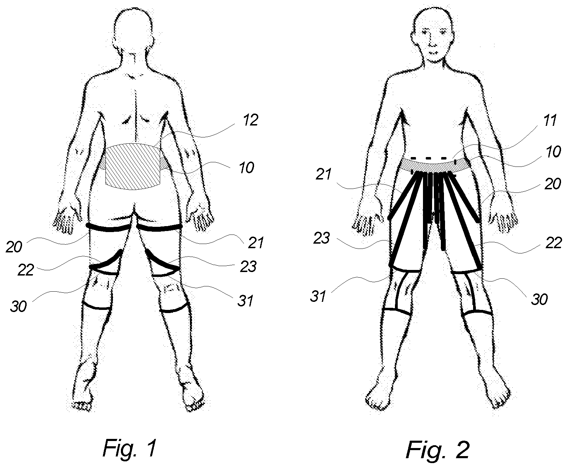

Figs. 1 and 2 illustrate a back support according to an embodiment of the invention. The user carries a waist band 10 comprising or connected to a supporting plate 12 exerting a pressure against the back of the user. In the shown embodiment, the plate 12 may provide excellent support for e.g. a damaged lower part of the user's spine. The pressure exerted against the back may be provided to a certain degree by the direct tightening of the waist band 10 but mainly by the pulling effect from a number of straps 20, 21, 22, 23 on the front of the user. This pulling effect is created by fastening both ends of the straps 20, 21, 22, 23 onto said waist band 10 within a fixing region 11 on the front side of the waist band 10, leading the straps around the legs as shown in the figures thereby leading the legs to provide a pulling force unto the waist band resulting in a pressure exerted onto the supporting plate against the back of the user.

In the shown embodiment two first leg straps 20, 21 are positioned on the upper region of the thighs and two second leg straps 22, 23 are positioned on the lower region of the thighs. Hereby all four straps may aid in providing an increased pressure exerted against the back. However, in another embodiment the back support may only comprise the two first leg straps (20, 21) arranged in the upper region of the thighs or the back support may only comprise the two second leg straps (22, 23) arranged in the lower region of the thighs.

The upper straps 20, 21 will normally remain in position as being restricted in movement by the crotch of the user, whereas the lower straps 22, 23 advantageously may be supported further to be held in position. Such further support is in the shown embodiment provided by a lower strap system 30, 31, which may be connected in different known ways to the lower straps 22, 23 thereby aiding them in maintaining the position on the legs of the user.

In the shown embodiment the strap system comprises two straps surrounding the legs, above and below the knees respectively, connected with a further strap. Obviously, the shape of the strap system may be varied a lot by the skilled person while still providing the desired extra support for the lower straps 22, 23. In another embodiment the position of the lower second leg straps 22, 23 or even the upper first leg straps 20, 21 could be substantially fixed on or in relation to the user's legs by means of a belt, a knee bandage, adhesive tape or other.

It should be noted that the position to be fixed or maintained by the straps 20, 21, 22, 23 on the legs of the user are primarily the lowest position of the respective straps 20, 21, 22, 23 - i.e. the above mentioned lower strap system 30, 31 would only fix the lower straps 22, 23 in the lowest position on the user's legs thereby leaving the rest of the length of the straps 22, 23 free to adapt to the user's current position, cloth and other.

It should also be noted that the attachments of the straps in the fixing region are shown very simplified in fig. 2. This attachment may be carried out according to conventional attachment methods, such as snap hooks, buttons, Velcro, tying a knot etc.

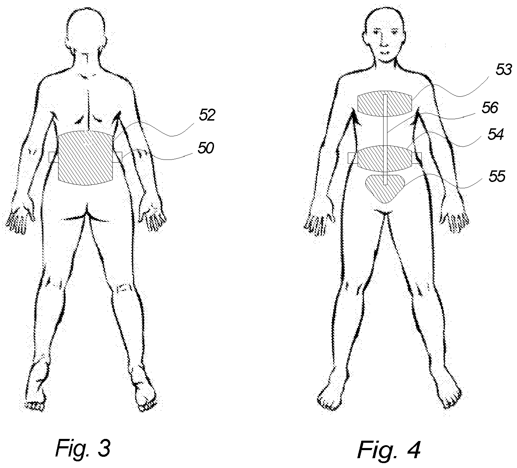

Figs. 3 and 4 illustrate a prior art back support. On the back a supporting plate 52 supports the back similarly to in the present invention. However, in this prior art back support, the pulling effect is created by a constant pressure on the front of the user exerted by three supporting plates 53, 54, 55 on the front, all three connected by a connecting guide 56. All elements of this prior art back support is rigid, also the connection 50 between the front and the back; consequently, this prior art back support holds the torso in a very fixed position, thereby making it unpleasant to wear.

It is noted that the curvature of the lower back of human beings is believed to facilitate the invention in that the back support when placed on the lower back will typically not be vertically placed on the back but instead have a forward inclination. Hereby the pressure required for the back support is pointed partly forwards and partly downwards. This may be provided by the straps according to embodiments of the present invention.

A major advantage of the present invention is the comfortability. As mentioned, the prior art back support is generally unpleasant to wear, which has the disadvantage that people tend to only wear it for limited time. Instead a back support according to embodiments of the present invention allows for prolonged use of a back support without discomfort. In preferred embodiments, the inventive back support may be worn daily/all day whereby the risk of recurrence in connection with back injuries may be minimized.

The back support according to the invention may be used anywhere, such as in hospitals, care homes, and private homes. It may be used with back damages and back problems as well as in adjusting posture or merely for comfort reasons.

## Claims

1. A back support comprising a waist band (10) with at least one front-facing fixing region (11) and at least one back-facing supporting plate (12) attached to said waist band; said at least one supporting plate being adapted to support the lower back region of the user,
**characterized in that**
said back support further comprises at least two first leg straps (20, 21, 22, 23) fixed in both ends unto said waist band in said fixing region; said first leg straps each forming a loop extending in use from the fixing region downwardly and behind a respective leg of the user and again upwardly to the fixing region;
said back support further comprises at least two second leg straps (20, 21, 22, 23) fixed in both ends unto said waist band in said fixing region; said second leg straps each forming a loop extending in use from the fixing region downwardly and behind a respective leg of the user and again upwardly to the fixing region; wherein said at least two first leg straps (20, 21) are adapted for, when in use, tightening behind the upper region of the thigh of the user, and said at least two second leg straps (22, 23) are adapted for, when in use, tightening behind the lower region of the thigh of the user.

2. A back support according to claim 1, wherein said supporting plate is substantially shaped as the shape of a back of a user to increase the comfortability for the user and/or said supporting plate comprises a pad to increase the comfortability for the user.

3. A back support according to claim 1 or 2, wherein said back support further comprises a connecting means for connecting said waist band to a fixed structure in front of the user, preferably said connecting means comprises a hook, a snap hook and/or a strap comprising a loop.

4. A back support according to claim 1 or 2, wherein said back support further comprises a lower strap system (30, 31) for, when in use, assisting said second leg straps (22, 23) in maintaining their position on the lower region of the thigh of the user; said lower strap system comprising at least one strap surrounding the leg of said user;
preferably said lower strap system, when in use, comprises two straps on each leg of said user, one above the knee and one below the knee, both surrounding the legs, and at least one connecting strap for connecting said straps on each leg.

5. A back support according to claim 1 or 2, wherein the straps are formed of an elastic material.

6. A back support according to claim 1 or 2, wherein said at least one back-facing supporting plate (12) has an area of more than 100cm², preferably more than 200cm².

7. A back support according to claim 1 or 2, wherein said at least one back-facing supporting plate (12) is fixed on said waist band or said waist band is connected to said supporting plate in each side of said supporting plate.

## Patentansprüche

1. Rückenstütze, die ein Taillenband (10) mit wenigstens einem nach vorne ausgerichteten Befestigungsbereich (11) und wenigstens einer nach hinten ausgerichteten Stützplatte (12), die an dem Taillenband befestigt ist, umfasst; wobei die wenigstens eine Stützplatte dafür eingerichtet ist, den unteren Rückenbereich des Benutzers zu stützen,
**dadurch gekennzeichnet, dass**
die Rückenstütze außerdem wenigstens zwei erste Beinbänder (20, 21, 22, 23) umfasst, die an beiden Enden in dem Befestigungsbereich auf dem Taillenband befestigt sind; wobei von den ersten Beinbändern jedes eine Schlaufe bildet, die sich während der Verwendung vom Befestigungsbereich nach unten und hinter ein zugehöriges Bein des Benutzers und wieder nach oben zum Befestigungsbereich erstreckt;
wobei die Rückenstütze außerdem wenigstens zwei zweite Beinbänder (20, 21, 22, 23) umfasst, die an beiden Enden in dem Befestigungsbereich auf dem Taillenband befestigt sind; wobei von den zweiten Beinbändern jedes eine Schlaufe bildet, die sich während der Verwendung vom Befestigungsbereich nach unten und hinter ein zugehöriges Bein des Benutzers und wieder nach oben zum Befestigungsbereich erstreckt; wobei die wenigstens zwei ersten Beinbänder (20, 21) dafür eingerichtet sind, während der Verwendung sich hinter dem oberen Bereich des Oberschenkels des Benutzers zu spannen, und wobei die wenigstens zwei zweiten Beinbänder (22, 23) dafür eingerichtet sind, während der Verwendung sich hinter dem unteren Bereich des Oberschenkels des Benutzers zu spannen.

2. Rückenstütze nach Anspruch 1, wobei die Stützplatte im Wesentlichen wie die Form eines Rückens eines Benutzers geformt ist, um den Komfort für den Benutzer zu erhöhen und/oder wobei die Stützplatte ein Polster umfasst, um den Komfort für den Benutzer zu erhöhen.

3. Rückenstütze nach Anspruch 1 oder 2, wobei die Rückenstütze außerdem ein Verbindungsmittel umfasst, um das Taillenband mit einer feststehenden Einrichtung vor dem Benutzer zu verbinden, wobei das Verbindungsmittel vorzugsweise Folgendes umfasst: einen Haken, einen Karabinerhaken und/oder ein Band, das eine Schlaufe umfasst.

4. Rückenstütze nach Anspruch 1 oder 2, wobei die Rückenstütze außerdem ein unteres Bandsystem (30, 31) umfasst, um während der Verwendung die zweiten Beinbänder (22, 23) darin zu unterstützen ihre Position auf dem unteren Bereich des Oberschenkels des Benutzers beizubehalten; wobei das untere Bandsystem wenigstens ein Band umfasst, das das Bein des Benutzers umschließt;
wobei vorzugsweise das untere Bandsystem während der Verwendung an jedem Bein des Benutzers zwei Bänder umfasst, eines oberhalb des Knies und eines unterhalb des Knies, wobei beide die Beine umschließen, sowie wenigstens ein Verbindungsband, um die Bänder an jedem Bein zu verbinden.

5. Rückenstütze nach Anspruch 1 oder 2, wobei die Bänder aus einem elastischen Material gebildet sind.

6. Rückenstütze nach Anspruch 1 oder 2, wobei die wenigstens eine nach hinten ausgerichtete Stützplatte (12) eine Fläche von mehr als 100 cm² und vorzugsweise von mehr als 200 cm² hat.

7. Rückenstütze nach Anspruch 1 oder 2, wobei die wenigstens eine nach hinten ausgerichtete Stützplatte (12) an dem Taillenband befestigt ist oder wobei das Taillenband mit der Stützplatte auf jeder Seite der Stützplatte verbunden ist.

## Revendications

1. Support dorsal comprenant une bande de taille (10) avec au moins une région de fixation orientée vers l'avant (11) et au moins une plaque de support orientée vers l'arrière (12) fixée à ladite bande de taille ; ladite au moins une plaque de support étant adaptée pour supporter la région du bas de dos de l'utilisateur,
**caractérisé en ce que** :
ledit support dorsal comprend en outre au moins deux premières sangle de jambe (20, 21, 22, 23) fixées dans deux extrémités à ladite bande de taille dans ladite région de fixation ; lesdites premières sangles de jambe comprenant chacune une boucle s'étendant, à l'usage, à partir de la région de fixation vers le bas et derrière une jambe respective de l'utilisateur et à nouveau vers le haut vers la région de fixation ;
ledit support dorsal comprend en outre au moins deux secondes sangles de jambe (20, 21, 22, 23) fixées dans deux extrémités à ladite bande de taille dans ladite région de fixation ; lesdites secondes sangles de jambe formant chacune une boucle s'étendant, à l'usage, à partir de la région de fixation vers le bas et derrière une jambe respective de l'utilisateur à nouveau vers le haut vers la région de fixation ; dans lequel lesdites au moins deux premières sangles de jambe (20, 21) sont adaptées pour, lorsqu'elles sont utilisées, serrer derrière la région supérieure de la cuisse de l'utilisateur, et lesdites au moins deux secondes sangles de jambe (22, 23) sont adaptées pour, lorsqu'elles sont utilisées, serrer derrière la région inférieure de la cuisse de l'utilisateur.

2. Support dorsal selon la revendication 1, dans lequel ladite plaque de support est sensiblement formée comme la forme du dos d'un utilisateur afin d'augmenter le confort pour l'utilisateur et/ou ladite plaque de support comprend un coussinet pour augmenter le confort pour l'utilisateur.

3. Support dorsal selon la revendication 1 ou 2, dans lequel ledit support dorsal comprend en outre un moyen de raccordement pour raccorder ladite bande de taille à une structure fixée à l'avant de l'utilisateur, de préférence ledit moyen de raccordement comprend un crochet, un mousqueton et/ou une sangle comprenant une boucle.

4. Support dorsal selon la revendication 1 ou 2, dans lequel ledit support dorsal comprend en outre un système de sangle inférieure (30, 31) pour, lorsqu'il est utilisé, aider lesdites secondes sangles de jambe (22, 23) à maintenir leur position sur la région inférieure de la cuisse de l'utilisateur ; ledit système de sangle inférieure comprenant au moins une sangle entourant la jambe dudit utilisateur ;
de préférence ledit système de sangle inférieure, lorsqu'il est utilisé, comprend deux sangles sur chaque jambe dudit utilisateur, une au-dessus du genou et une au-dessous du genou, entourant toutes deux les jambes, et au moins une sangle de raccordement pour raccorder lesdites sangles sur chaque jambe.

5. Support dorsal selon la revendication 1 ou 2, dans lequel les sangles sont formées avec un matériau élastique.

6. Support dorsal selon la revendication 1 ou 2, dans lequel ladite au moins une plaque de support orientée vers le dos (12) a une surface supérieure à 100 cm², de préférence supérieure à 200 cm².

7. Support dorsal selon la revendication 1 ou 2, dans lequel ladite au moins une plaque de support orientée vers le dos (12) est fixée sur ladite bande de taille ou bien ladite bande de taille est raccordée à ladite plaque de support de chaque côté de ladite plaque de support.
